# EUROPÄISCHE PATENTSCHRIFT

(11) **EP 1 046 638 B1**
(45) Veröffentlichungstag und Bekanntmachung des Hinweises auf die Patenterteilung: **01.10.2003**
(21) Anmeldenummer: 99117122.4
(22) Anmeldetag: 31.08.1999
(51) Int. Cl.: C07C 269/00, C07C 273/18, C07C 271/22, C07C 275/16, C07C 275/24

(54) **Verfahren zur Herstellung von Aminosäurederivaten**
Process for the preparation of aminoacid derivatives
Procédé pour la préparation de dérivés d'aminoacides

(30) Priorität: 21.04.1999 DE 19917961
(43) Veröffentlichungstag der Anmeldung: 25.10.2000
(73) Patentinhaber: Degussa AG, 40474 Düsseldorf (DE)
(72) Erfinder: Kleemiss, Wolfgang, Dr., 45721 Haltern (DE); Feld, Marcel, Dr., 51147 Köln (DE)

(56) Entgegenhaltungen:
- CHEMICAL ABSTRACTS, vol. 38, no. 2, 20. Januar 1944 (1944-01-20) Columbus, Ohio, US; LIANG LI ET AL.: "VI. Synthesis of alpha-amino-ethylpropylacetic acid" Spalte 335; XP002147330 & J. CHINESE CHEM. SOC., Bd. 9, 1942, Seiten 14-30,
- W. TREIBS ET A.: "Synthesen mit Dicarbonsäuren, XIX. Mitteil.: Darstellung von omega-Aminocarbonsäuren durch halbseitigen Hoffmanschen Abbau von Dicarbonsäuren" BERICHTE DER DEUTSCHEN CHEMISCHEN GESELLSCHAFT., Bd. 89, Nr. 1, 1956, Seiten 117-120, XP002147329 WEINHEIM DE

## Beschreibung

Die Erfindung betrifft ein Verfahren zur Herstellung von Aminosäurederivaten der allgemeinen Formel I 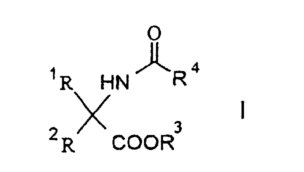 wobei R¹ C₁₋₆-Alkyl oder C₃₋₆-Cycloalkyl und R² C₁₋₆-Alkyl, Phenyl, Heteroaryl-, (CH₂)₁₋₃-Phenyl oder -(CH₂)₁₋₄-COOR bedeutet oder R¹ und R² zusammen -(CH₂)₂₋₆, -(CH₂)₂-O-(CH₂)₂- oder 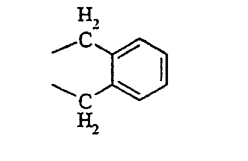 bedeuten,
oder R¹ Wasserstoff und R² einen tertiären Kohlenwasserstoffrest mit 4 bis 10 C-Atomen bedeuten,
R³ Wasserstoff oder einen Alkylrest mit 1-4, insbesondere 1 oder 2 Kohlenstoffatomen und R⁴ OR, wobei R in R² und R⁴ für einen aliphatischen, aromatischen oder araliphatischen Rest mit 1 bis 8 C-Atomen steht, oder NR'R", wobei R' für Wasserstoff oder für einen aliphatischen, aromatischen oder araliphatischen Rest mit 1 bis 8 C-Atomen und R" für Wasserstoff oder einen gegebenenfalls von R' verschiedenen aliphatischen, aromatischen oder araliphatischen Rest mit 1 bis 8 C-Atomen steht, bedeutet,
aus den entsprechenden Malonsäuremonoesteramiden der allgemeinen Formel II durch Hofmannschen Abbau mittels eines Hypohalits in wäßrig basischem Milieu in Gegenwart eines Alkohols oder Amins 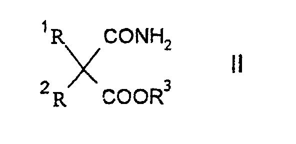 wobei R¹, R² und R³ die oben angegebenen Bedeutungen haben.

Wenn R¹ von R² verschieden ist, sind sowohl die Verbindungen der allgemeinen Formel II, als auch die Verbindungen der allgemeinen Formel I chiral. Man erhält also aus den Racematen der Verbindungen der allgemeinen Formel II auch die Racemate der Aminosäurederivate der allgemeinen Formel I. Andererseits erhält man aus den entsprechenden enantiomerenreinen Verbindungen der allgemeinen Formel II, mit R¹ ungleich R², auch die entsprechenden enantiomerenreinen Verbindungen der allgemeinen Formel I.

Aminosäuren-und deren Derivate besitzen eine große Bedeutung für die Synthese von pharmakologisch aktiven Verbindungen (A. S. Bommarius, M. Schwarm und K Drauz, J. Mol. Catalysis B: Enzymatic 5 (1998) 1-11). Neben den natürlich vorkommenden Aminosäuren nimmt auch die Bedeutung der unnatürlichen Aminosäuren und deren Derivate zu. So werden für die Synthese von hochwirksamen Pharmaprodukten Derivate von speziellen unnatürlichen Aminosäuren benötigt. Die Erarbeitung von geeigneten Syntheseverfahren für die Herstellung von speziellen Aminosäuren ist also von großem Interesse.

Unter den unnatürlichen Aminosäuren besitzen die 2,2-Dialkylaminoessigsäuren als unnatürliche Aminosäuren eine große Bedeutung für die Synthese von speziellen Peptiden. Die im Vergleich zu den natürlichen Aminosäuren zusätzliche Alkylgruppe in 2-Stellung führt zu einer konformativen Starrheit der entsprechenden Peptidbindung und beeinflußt dadurch maßgeblich die Tertiärstruktur des Gesamtpeptids (D. Obrecht, M. Altorfer, U. Bohdal, J. Daly, W. Huber, A. Labhardt, C. Lehmann, K. Muller, R. Ruffieux, P. Schonholzer, C. Spiegler, C. Zumbnmm, Biopolymers 42(5), 575-626 (1997); M. Crisma, G. Valle, M. Pantano, F. Formaggio, G. M. Bonora, C. Toniolo, J. Kamphius, Recl. Trav. Chim. Pays-Bas 114(7), 325-31 (1995); S. Prasad, B. R. Rao, P. Balaram, Biopolymers 35(1), 11-20, (1995)).
2,2-Dialkylaminoessigsäuren wurden ebenfalls als lipophile Aminosäuren für den Aufbau von Peptiden eingesetzt, die als potentielle Wirkstoffkandidaten in Frage kommen (P. M. Hardy, I. N. Lingham, Int. J. Peptide Protein Res. 21, 392-405 (1983)).

In der Europäischen Patentanmeldung EP-A 770 613 ist darüberhinaus die Synthese von 5,5-disubstituierten Imidazolidin-2,4-dionen, die als Immunodilatoren eingesetzt werden, beschrieben. Bei ihrer Synthese geht man von den entsprechenden 2,2-Dialkylaminosäureestern aus.

Substituierte Hydantoin-Verbindungen, die einfach aus den Verbindungen der allgemeinen Formel I mit R⁴ = OR, wobei R für einen aliphatischen, aromatischen oder araliphatischen Rest mit 1 bis 8 C-Atomen steht, hergestellt werden können (B.A. Dressmann, L.A. Spangle, W. Kaldor, Tetrahedron Lett. 937-940 (1996)) sind generell als Pharma-Produkte oder -Vorprodukte von großer Bedeutung. Im pharmazeutischen Bereich sind für Hydantoin-Verbindungen insbesondere antikonvulsive, antiinflammatorische (J. Med. Chem. 8, 239 (1965); Arzneim. Forsch./ Drug Res. 27(II), 1942 (1977); Pharmazie 38, 341 (1983), J. Med.Chem. 28, 601 (1985)) und Antitumorwirkungen (J. Med. Chem. 18, 846 (1975); Arzneim. Forsch./Drug Res. 34(I), 663 (1984)) bekannt.

Die Synthese von 2,2-Di-n-propylaminoessigsäureethylester wurde ausgehend von Cyanessigsäureethylester beschrieben (J. Chinese Chem. Soc. 8, 81-91 (1941)) (Schema 1).

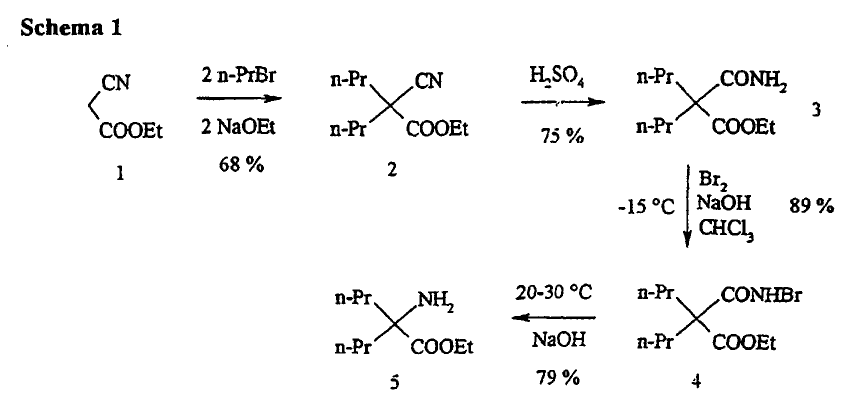

Dabei überführte man den Cyanessigsäureethylester zunächst mit Natriumethylat in das Enolat und setzte dann mit 2 Äquivalenten Propylbromid um. Der in einer Ausbeute von 68 % resultierende 2,2-Di-n-propylcyanessigsäureethylester **2** wurde dann mit Schwefelsäure in einer Ausbeute von 75 % in den 2,2-Di-n-propylmalonsäuremonoamidethylester 3 überführt. Durch Umsetzung mit einem Gemisch aus Natronlauge und Brom in Chloroform erhielt man dann in einer Ausbeute von 89 % den 2,2-Di-n-propyl-N-brom-malonsäuremonoamidethylester **4**. Dieser wurde mit einem Überschuß an Natronlauge durch Hofmann-Abbau in einer Ausbeute von 79 % in die Zielverbindung **5** überführt. Die Gesamtausbeute ausgehend von Cyanessigsäureethylester betrug also 36 %.

Die Synthese dieser Verbindung, die auch als Di-n-propylglycinethylester bezeichnet werden kann, wurde später optimiert (P. M. Hardy, I. N. Lingham, Int. J. Peptide Protein Res. 21, 392-405 (1983)). Die Synthese von 2,2-Di-n-propylcyanessigsäureethylester **2** gelang in einer Ausbeute von 81%, während die nachfolgende Umsetzung zum 2,2-Di-n-propylmalonsäuremonoamidethylester **3** eine Ausbeute von 82 % ergab. Nach der weiteren Reaktion mit Brom und NaOH in CHCl₃ bei tiefer Temperatur wurde nicht das N-Bromamid **4** isoliert, sondern nach Behandlung mit einem 4-fachen Überschuß Natronlauge das entsprechende Isocyanat **6** in einer destillativen Ausbeute von 81 % erhalten. Dieses wurde dann mit einem Überschuß an 3M HCl unter Rückfluß gekocht. Anschließendes Alkalisieren mit Natronlauge ergab den Aminoester **5** in einer Ausbeute von 87 %. Die Gesamtausbeute dieser Synthese betrug ausgehend vom Cyanessigester **1** ca. 47 %.

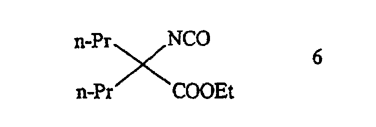

Hardy et al. betonen, daß die Isolierung des Isocyanats und die anschließende saure Verseifung zum Aminoester **5** von großem Vorteil ist, da bei dieser Vorgehensweise die Entstehung eines bei der Aufarbeitung sehr störenden Harnstoffderivates, **7** vermieden wird. Unter alkalischen Bedingungen reagiert nämlich ein Teil des Reaktionsproduktes **5** mit dem Isocyanat **6** zu dem störenden Harnstoffderivat 7, was die Ausbeute außerdem erheblich vermindert.

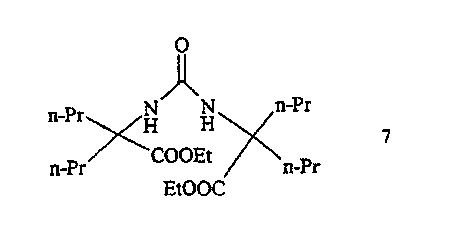

Trotz der höheren Ausbeute, die von Hardy et al. mit ihrer Synthesestrategie erreicht wurde, beinhaltet das Verfahren nach Schemal eine Reihe von Problemen. So ist die Durchführung des Hofinann-Abbaus in Chloroform mit einem Gemisch aus Brom und Natronlauge sicher nur als Labormethode geeignet. Auch ist eine Reaktionstemperatur von -15 °C in der Technik nur mit großem Aufwand zu gewährleisten. Ein weiterer Nachteil des Verfahrens ist die Notwendigkeit der Isocyanat-Isolierung, um die Bildung des Harnstoffderivates 7 zu verhindern. Dies ist erstens ein zusätzlicher Schritt und erfordert zweitens die anschließende saure Verseifung zum Aminosäureester **5**. Hierbei fällt wieder Salz in stöchiometrischen Mengen an, da das Reaktionsgemisch vor der Extraktion des Produktes alkalisiert werden muß.

Vor dem Hintergrund des großen Syntheseaufwandes für die Herstellung von **5** ist die Gesamtausbeute von insgesamt 47 % ausgehend von Cyanessigester **1** also nicht zufriedenstellend. Die Ausbeute der Hofinann-Abbau-Reaktion von **3** nach **5** beträgt ca. 70 %.

Eine weitere Methode zur Herstellung des Aminoesters **5** geht aus von racemischem Norvalin, einer unnatürlichen Aminosäure (EP 770613) (Schema 2).

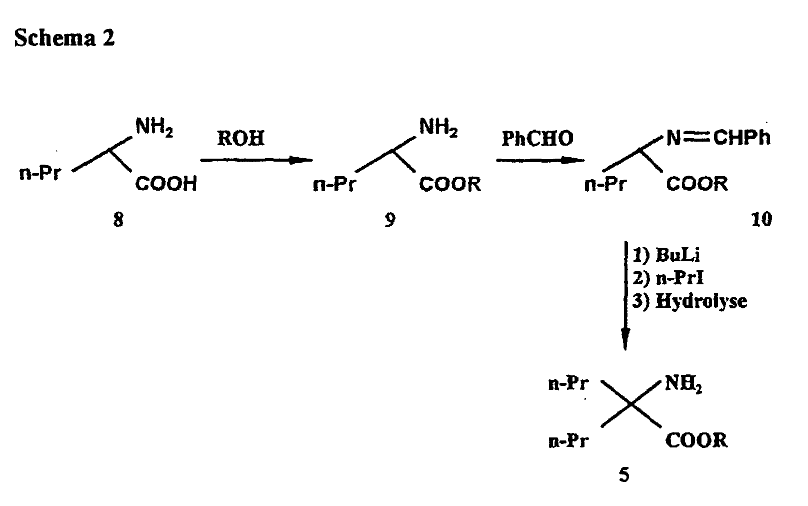

Dabei wird zunächst die Aminosäure in den Ester überführt. Anschließend wird durch Reaktion mit Benzaldehyd ein Imin synthetisiert, das durch Deprotonierung mit n-Butyllithium und Alkylierung mit Propyliodid mit anschließender Hydrolyse zum Di-n-propylglycinester umgesetzt wird. Die Gesamtausbeute über alle Stufen beträgt nur 53 %.

Abgesehen von der Tatsache, daß die Ausgangsverbindung der Synthese, Norvalin, deutlich teurer ist als Cyanessigester, sind die Einzelschritte, bis auf die Veresterungstufe, in der Technik nur schwer zu realisieren bzw. mit sehr hohen Kosten verbunden. In der zweiten Stufe wird in stöchiometrischen Mengen Benzaldehyd verwandt, der nach der Alkylierung wieder abgespalten werden muß.

Die Alkylierung wird außerdem mit dem teuren Propyliodid durchgeführt. Desweiteren ist für die Alkylierung n-Butyllithium als Base notwendig. Diese Base ist aber erstens teuer und zweitens in der Technik nur unter großen Sicherheitsvorkehrungen zu handhaben.

Ein weiteres Problem, das für beide in den Schemata 1 und 2 beschriebenen Synthesen gilt, ist die Generierung von großen Abfallsalzmengen. Bei der Durchführung dieser Synthesen im großen Maßstab führt die Entsorgung des Abfallsalzes zu hohen Kosten. In dem Verfahren nach dem Schema 2 muß darüber hinaus der Salzanfall berücksichtigt werden, der bei der Synthese des Ausgangsmaterials **8** entsteht.

Es bestand daher die Aufgabe, ein Verfahren zur Herstellung von Aminosäurederivaten der allgemeinen Formel I, aus denen man in einfacher Weise die entsprechenden Aminosäurederivate mit freier Aminogruppe oder die Aminosäuren selbst herstellen kann, bereitzustellen, das, von wohlfeilen Ausgangsstoffen ausgehend, gute Ausbeuten und Raum/Zeit-Ausbeuten ergibt, keine sicherheitstechnisch anspruchsvollen Stoffe erfordert und eine möglichst einfache Aufarbeitung des Produktes ohne eine aufwendige Abtrennung von Reaktionssalzen ermöglicht.

Erfindungsgemäß wird diese Aufgabe gelöst und werden Aminosäurederivate der allgemeinen Formel I vorteilhaft dadurch hergestellt, daß man Malonsäuremonoesteramide der allgemeinen Formel II in einem Hofmann-Abbau mit insbesondere 1,0 bis 1,2 Äquivalenten einer Hypohalit-Lösung und 1,0 bis 4,0 Äquivalenten eines Alkalihydroxids in Gegenwart eines aliphatischen, aromatischen oder araliphatischen Alkohols mit 1 bis 8 C-Atomen oder eines aliphatischen, aromatischen oder araliphatischen primären oder sekundären Amins mit 1 bis 16 C-Atomen oder Ammoniak, umsetzt.

Gegenstand der Erfindung ist daher ein Verfahren zur Herstellung von Aminosäurederivaten der allgemeinen Formel I 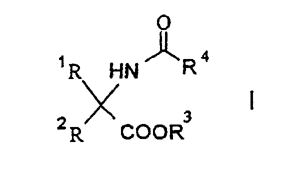 wobei R¹ C₁₋₆-Alkyl oder C₃₋₆-Cycloalkyl und R² C₁₋₆-Alkyl, Phenyl, Heteroaryl-, (CH₂)₁₋₃-Phenyl oder -(CH₂)₁₋₄-COOR bedeutet oder R¹ und R² zusammen -(CH₂)₂₋₆, -(CH₂)₂-O-(CH₂)₂- oder 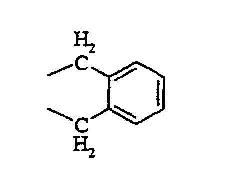 bedeuten,
oder R¹ Wasserstoff und R² einen tertiären Kohlenwasserstoffrest mit 4 bis 10 C-Atomen bedeuten,
R³ Wasserstoff oder einen Alkylrest mit 1-4, insbesondere 1 oder 2 Kohlenstoffatomen und R⁴ OR, wobei R in R² und R⁴ für einen aliphatischen, aromatischen oder araliphatischen Rest mit 1 bis 8 C-Atomen steht, oder NR'R", wobei R' für Wasserstoff oder für einen aliphatischen, aromatischen oder araliphatischen Rest mit 1 bis 8 C-Atomen und R" für Wasserstoff oder einen gegebenenfalls von R' verschiedenen aliphatischen, aromatischen oder araliphatischen Rest mit 1 bis 8 C-Atomen steht, bedeutet,
aus den entsprechenden Malonsäuremonoesteramiden der allgemeinen Formel II 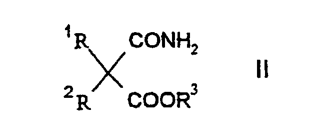 wobei R¹, R² und R³ die oben angegebenen Bedeutungen haben, durch Hofmannschen Abbau mittels eines Hypohalits in wäßrig basischem Milieu, dadurch gekennzeichnet, daß man die Reaktion in Gegenwart eines Alkohols oder Amins durchführt.

Üblicherweise resultieren nach dem Hofmann-Abbau von substituierten Malonesteramiden direkt die freien Aminosäuren in Form ihrer Salze (EP-A 0676 390, DE 197 24 086). Diese sind sehr gut in Wasser löslich. Die Aminosäuren müssen dann in aufwendigen Extraktionsverfahren oder Ionenaustauschverfahren vom ebenfalls wasserlöslichen Reaktionssalz abgetrennt werden.

Bei der erfindungsgemäßen Reaktionsführung entstehen neben kleinen Mengen der entsprechenden Aminosäuren oder deren Ester die entsprechenden N-Alkoxycarbonylaminosäuren bzw.N-Carbamoylaminosäuren oder deren Ester. Diese Verbindungen können nach der Entfernung des Alkohol- bzw. Amin-Überschusses und gegebenenfalls nach Ansäuren des resultierenden Reaktionsgemisches durch Extraktion mit einem organischen Lösungsmittel isoliert werden.

Die Tatsache, daß der Hofmann-Abbau von Amiden mit Alkalihypohalit in Gegenwart von Alkoholen zu den entsprechenden Carbamaten führt, ist an sich bekannt (E. S. Wallis, J. F. Lane, Organic Reactions III, [1946], 267-306). Die Anwendung dieser Reaktionsfahrweise auf substituierte Malonsäuremonoamidester der allgemeinen Formel I ist jedoch neu. Überraschend ist, daß je nach Substitutionsmuster am Malonat-C-Atom der Hofmann-Abbau von Verbindungen der allgemeinen Formel II in Gegenwart eines Alkohols oder Amins neben kleinen Mengen der entsprechenden Aminosäuren oder deren Ester entweder zur freien N-Alkoxycarbonylaminosäure, bzw. N-Carbamoylaminosäure oder zu den entsprechenden Estern führt. Die Ester können nach destillativer Entfernung des Alkohol- bzw. Amin-Überschusses durch Extraktion mit Hilfe eines organischen Lösungsmittels gewonnen werden, während die freien Säuren erst nach Ansäuerung des vom Lösungsmittel befreiten Reaktionsaustrags durch Extraktion mit einem organischen Lösungsmittel (z. B. Methyl-tert-butylether, Diethylether, Toluol, Cyclohexan) gewonnen werden können.

Es entfällt also eine aufwendige Trennung von zusammen in der wäßrigen Phase vorliegendem Reaktionsprodukt und Reaktionssalzen durch Ionenaustauschchromatographie oder spezielle Membranverfahren.

Bei den herkömmlichen Verfahren zur Herstellung von Aminosäuren (Strecker-Synthese, sämtliche biotechnologische Verfahren und andere) fallen Aminosäure und Reaktionssalz zusammen in wäßriger Lösung an. Hier sind aufwendige Verfahren zur Trennung des Reaktionsproduktes vom Reaktionssalz notwendig.

Ein weiterer Vorteil des erfindungsgemäßen Verfahrens ist, daß die Verwendung eines sehr viel geringeren Alkali-Überschusses als von Hardy et al. eingesetzten zu guten Ausbeuten der N-Alkoxycarbonylaminosäuren bzw. N-Carbamoylaminosäuren oder deren entsprechender Ester führt.

Bereits der Einsatz von einem Äquivalent Alkalihydroxyd bezogen auf den Malonsäuremonoamidester der allgemeinen Formel II führt zu guten Ausbeuten von Verbindungen der allgemeinen Formel I.

Die direkte Bildung der N-Alkoxycarbonylaminosäuren, bzw. N-Carbamoylaminosäuren oder deren entsprechender Ester ist auch insofern sehr interessant, als man im Pharma- und Agrobereich nach Synthesemethoden sucht, mit denen man auf einfache Weise Derivate von speziellen Aminosäuren herstellen kann, die wiederum für eine Racematspaltung in die Enantiomere zu verwenden sind. Hierbei handelt es sich z. B. um die N-Acylaminosäuren (A. S. Bommarius, K. Drauz, K. Günther, G. Knaup und M. Schwarm, Tetrahedron: Asymmetry 3197-3200 (1997)) oder um die N-Alkoxycarbonylderivate der entsprechenden Aminosäuren (DE 36 06 401; Ann. N. Y. Acad. Sci (Enzyme 9), 343-345 (1988)).

Für den Hofmannschen Abbau werden eine Base und ein Hypohalit, d.h. ein Salz einer unterhalogenigen Säure, benötigt. Von den Hypohalitlösungen werden zweckmäßig die gut zugänglichen , wohlfeilen Hypochlorite eingesetzt. Die bevorzugten Hypochlorite sind Alkalihypochlorite wie Kaliumhypochlorit und insbesondere Natriumhypochlorit in Form ihrer wäßrigen Lösung, die auch als Bleichlauge bezeichnet wird. Calciumhypochlorit ist ebenfalls verwendbar, gibt aber geringere Ausbeuten. Das Hypohalit wird in einer Menge von 1,0 bis 1,5, vorzugsweise 1,0 bis 1,2 Äquivalenten, bezogen auf das Edukt II, angewandt.

Bevorzugte Basen sind die Alkalihydroxide, wie Kaliumhydroxid und insbesondere Natriumhydroxid, wieder in Form ihrer wäßrigen Lösungen. Auch Erdalkalihydroxide sind geeignet, ergeben aber geringere Ausbeuten. Im allgemeinen verwendet man eine Base mit dem Kation, das auch im Hypohalit vorliegt. Die Base wird in einer Menge von 0,8 bis 4,0, vorzugsweise 0,8 bis 1,5 Äquivalenten je Äquivalent des Edukts II eingesetzt.

Die Umsetzung findet im wäßrig basischen Milieu in Gegenwart eines 2 bis 20-fachen molaren Überschusses des Alkohols oder Amins statt. Im allgemeinen entfallen auf das Wasser 30 bis 80, insbesondere 40 bis 80 Gew.-% des Reaktionsgemisches.

Als Alkohole können primäre, sekundäre oder tertiäre Alkohole mit 1 bis 8 C-Atomen, wie z.B. Methanol, Ethanol, Isopropanol, t-Butanol, 2-Ethylhexanol, Benzylalkohol, etc. verwandt werden. Neben Ammoniak können primäre oder sekundäre Amine mit 1 bis 16 C-Atomen, wie z. B. Diethylamin, Di-n-propylamin, Butylamin oder Benzylamin verwandt werden.

Das erfindungsgemäße Verfahren kann kontinuierlich oder diskontinuierlich durchgeführt werden. Bei einer diskontinuierlichen Ausführung mit Alkalihydroxid wie z. B. Natriumhydroxid als Base und einem Hypochlorit wie z. B. Natriumhypochlorit als Salz einer unterhalogenigen Säure in Gegenwart eines Alkohols oder eines Amins setzt man zunächst bei einer Temperatur, die im allgemeinen 0 bis 20 °C, vorzugsweise 5 bis 10 °C, beträgt mit einer Reaktionszeit von 0,25 bis 10 Stunden, vorzugsweise 0,5 bis 4 Stunden, um und erwärmt das resultierende Reaktionsgemisch auf eine Temperatur von 40 bis 100 °C, vorzugsweise von 60 bis 80 °C. Je nach Temperatur ist die Reaktion nach 2 Minuten bis 2 Stunden beendet.

Nach dem erfindungsgemäßen Verfahren kann das Edukt der allgemeinen Formel II auch zunächst nur mit der wäßrigen Hypochlorit-Lösung bei einer Temperatur von 0 bis 20 °C, vorzugsweise 5 bis 10 °C, 0,5 bis 4 Stunden lang umgesetzt werden und nach Zugabe des Alkalihydroxids anschließend dieses kalte Reaktionsgemisch in einen 2 bis 20-fachen Überschuß eines Alkohols oder eines Amins, gegebenenfalls in wäßriger Lösung bei einer Temperatur von 40 bis 100 °C, vorzugsweise 60 bis 80 °C, eingetragen werden.

Nach dem Abkühlen des Reaktionsgemisches wird das Produkt I gegebenfalls nach vorheriger destillativer oder extraktiver Abtrennung des Alkohols oder Amins als organische Phase abgetrennt. Die wäßrige, untere Phase kann mit Hilfe eines inerten organischen Lösungsmittels extrahiert werden, um weiteres Produkt zu gewinnen. Die vereinigten organischen Phasen können nach dem Trocknen über z. B. Molekularsieb oder wasserbindenden Salzen (Magnesiumsulfat, Natriumsulfat usw.) von organischem Lösungsmittel befreit werden. Man erhält so das rohe Produkt I, das durch Destillation und/ oder Extraktion mit einem inerten organischen Lösungsmittel gereinigt werden kann. Man erhält so das Produkt mit hoher Reinheit und in einer Ausbeute von > 80 %. Um weiteres Rohprodukt der allgemeinen Formel I mit R³ = H zu erhalten, kann man die verbliebene wäßrige Phase ansäuern und nochmals mit einem inerten organischen Lösungsmittel erschöpfend extrahieren.

Die erfindungsgemäße Reaktionsfahrweise verhindert weitestgehend die Bildung unerwünschter Harnstoffderivate, die der Verbindung der Formel 7 analog sind. Überraschenderweise wird die störende Dimerisierungsreaktion zu den entsprechenden Harnstoffderivaten unter den erfindungsgemäßen Bedingungen zurückgedrängt.

Es ist also auf diese Weise möglich, die N-Alkoxycarbonylaminosäuren, bzw. N-Carbamoylaminosäuren oder deren entsprechende Ester der allgemeinen Formel I in hoher Ausbeute und mit hoher Selektivität herzustellen.

Es entfällt dabei die Notwendigkeit, das instabile Isocyanat analog zur Formel **6** zu isolieren und in einem gesonderten Schritt die Hydrolyse zum Endprodukt in konzentrierter Salzsäure durchzuführen.

Die Überführung von aromatischen oder aliphatischen Amiden in die Methylcarbamate durch Hofmann-analoge Reaktionsbedingungen ist zwar prinzipiell beschrieben (W. Keillor et al. J. Org. Chem. 62, 7495-7496 (1997). Doch es handelt sich ausnahmslos um Bedingungen, die nur für den Labormaßstab geeignet sind. So wird das entsprechende Amid mit zwei Äquivalenten N-Bromsuccinimid in Gegenwart einer Amidinbase und mit Methanol als Lösungsmittel in Ausbeuten von 40 bis maximal 95 % zum entsprechenden Methylcarbamat umgesetzt. Keillor et al. haben die Umsetzung von substituierten Malonsäuremonoesteramiden der allgemeinen Formel II mit N-Brom-succinimid zu den entsprechenden Methylcarbamaten der entsprechenden Aminoessigsäureester der allgemeinen Formel I nicht beschrieben.

N-Brom-succinimid ist sehr viel teurer als Alkalihypohalit, insbesondere Natriumhypochlorit. Außerdem resultieren unter den von Keillor et al. genannten Reaktionsbedingungen 2 Äquivalente Succinimid, die entsorgt werden müssen. In der Technik wären die Entsorgungskosten für das Succinimid nicht tragbar.
Bei dem erfindungsgemäßen Verfahren hingegen entsteht aus dem Alkalihypohalit, insbesondere Natriumhypochlorit, das Alkalihalogenid, insbesondere Natriumchlorid in Form seiner wäßrigen Lösung. Diese kann problemlos entsorgt werden.

Carbamate der allgemeinen Formel I sind wiederum sehr einfach in die entsprechenden Aminosäuren überführbar (Houben Weyl 15/1, 117 (1974); C. Cativiela, M. D. Diaz-de-Villegas, J. A. Gälvez, Y. Lapena, Tetrahedron 51, 5921-28 (1995)). N-Carbamoylaminoessigsäurederivate der allgemeinen Formel I, worin R⁴ NR'R" bedeutet und R' für einen aliphatischen, aromatischen oder araliphatischen Rest mit 1 bis 8 C-Atomen und R" für Wasserstoff oder einen gegebenenfalls von R' verschiedenen aliphatischen, aromatischen oder araliphatischen Rest mit 1 bis 8 C-Atomen steht, sind aber auch hochinteressante Ausgangsverbindungen für die Synthese von substituierten Hydantoinen, wie sie z. B in EP-A-0 770 613 beschrieben sind.

N-Alkoxycarbonylaminosäuren, bzw. N-Carbamoylaminosäuren oder deren entsprechende Ester der allgemeinen Formel I sind natürlich auch ausgehend von den entsprechenden Aminosäuren zugänglich. Doch hierzu muß die betreffende Aminosäure einfach zugänglich sein. Außerdem muß dann die Aminosäure oder ihr Ester mit Phosgen oder einem Phosgenderivat und anschließend mit Alkohol oder Amin umgesetzt werden (Houben Weyl 15/1, 46 (1974)). Phosgen ist hochgiftig und kann nur unter großen Sicherheitsvorkehrungen gehandhabt werden. Auch vor diesem Hintergrund hat das erfindungsgemäße Verfahren große Vorteile, da N-Alkoxycarbonylaminosäuren, bzw. N-Carbamoylaminosäuren oder deren entsprechende Ester der allgemeinen Formel I ohne die Verwendung von hochgiftigem Phosgen oder Phosgenderivaten hergestellt werden können.

Die folgenden Beispiele sollen das Verfahren der Erfindung weiter erläutern, nicht aber seinen Schutzbereich begrenzen, wie er in den Patentansprüchen definiert ist.

### Beispiel 1

In einem Rührgefäß werden 60,0 g (0,28 mol) 2,2-Di-n-propylmalonsäuremonoethylesteramid in 300 g Methanol gelöst. Zu der Lösung, die auf 5 °C abgekühlt worden ist, gibt man 58,0 g (0,29 mol) 20 %ige Natronlauge und 263,4 g (0,33 mol) 9,3 % ige Natriumhypochloritlösung derart hinzu, daß die Temperatur des Reaktionsgemisches 10 °C nicht übersteigt. Man läßt dann 1h bei ca. 5 °C rühren und dosiert anschließend die erhaltene Suspension (während der Chlorierung fällt anorganisches Salz aus) kontinuierlich über eine Fritte, um das Salz abzutrennen. Das klare Filtrat wird kontinuierlich durch einen auf 68 °C beheizten Rohrreaktor dosiert. Man wählt das Volumen des Rohrreaktors und die Dosiermenge pro Zeit so, daß im Rohrreaktor eine Verweilzeit des Filtrats von 2 bis 3 Minuten gewährleistet ist. Nachdem das Filtrat durch den Reaktor dosiert worden ist, wird noch 200 ml Methanol hindurchgepumpt. Der Reaktionsaustrag wird bei einem Druck von 40 mbar und einer Temperatur von 40 °C vom Methanol befreit. Der Rückstand wird erschöpfend mit Methyl-t-butylether (MTBE) extrahiert. Die organischen Phasen werden vereinigt und getrocknet. Anschließend wird das Lösungsmittel abdestilliert. Zurück bleiben 57,3 g eines Öls, das laut 13C-NMR-Spektrum zu >97 % aus N-Methoxycarbonyl-2,2-di-n-propylaminoessigsäureethylester (81 % Ausbeute) besteht. Nebenbestandteil ist 2,2-Di-n-propylaminoessigsäureethylester (3 % Ausbeute).

Die Aminosäure Di-n-propylglycin kann in Form ihres Hydrochlorids erhalten werden, indem der N-Methoxycarbonyl-2,2-di-n-propylaminoessigsäureethylester und der 2,2-Di-n-propylaminoessigsäureethyl-ester vereinigt werden und das Gemisch mit konzentrierter Salzsäure für 12 Stunden unter Rückfluß gekocht wird. Nach dem Abkühlen extrahiert man mit MTBE und engt die wäßrige Lösung zur Trockne ein. Man nimmt nochmals in Wasser auf und engt die Lösung im Vakuum ein, um überschüssige Salzsäure zu entfernen. Zurück bleiben 43,8 g (80 %) Di-n-propylglycinhydrochlorid. Hieraus läßt sich nach dem Stand der Technik die freie Aminosäure erhalten.

### Beispiel 2

In einem Rührgefäß werden 60,0 g (0,32 mol) 2,2-Diethylmalonsäuremonoethylesteramid in 300 g Methanol gelöst. Zu der Lösung, die auf 5 °C abgekühlt worden ist, gibt man 65,0 g (0,33 mol) 20 % ige Natronlauge und 280,2 g (0,35 mol) 9,3 % ige Natriumhypochloritlösung derart hinzu, daß die Temperatur des Reaktionsgemisches 10 °C nicht übersteigt. Man läßt dann 1h bei ca. 5 °C rühren und dosiert anschließend die erhaltene Suspension (während der Chlorierung fällt anorganisches Salz aus) kontinuierlich über eine Fritte, um das Salz abzutrennen. Das klare Filtrat wird kontinuierlich durch einen auf 68 °C beheizen Rohrreaktor dosiert. Man wählt das Volumen des Rohrreaktors und die Dosiermenge pro Zeit so, daß im Rohrreaktor eine Verweilzeit des Filtrats von 2 bis 3 Minuten gewährleistet ist. Nachdem das Filtrat durch den Reaktor dosiert worden ist, wird noch 200 ml Methanol hindurchgepumpt. Der Reaktionsaustrag wird bei einem Druck von 40 mbar und einer Temperatur von 40 °C vom Methanol befreit. Der Rückstand wird erschöpfend mit MTBE extrahiert. Die organischen Phasen werden vereinigt und getrocknet. Anschließend wird das Lösungsmittel abdestilliert. Zurückbleiben 49,1 g eines Öls, das laut 13C-NMR-Spektrum zu >85 % aus N-Methoxycarbonyl-2,2-diethylaminoessigsäureethylester (60 % Ausbeute) besteht. Nebenbestandteil 2,2-Diethylaminoessigsäureethylester (12 %).

Die nach der Extraktion zurückbleibende wäßrige, alkalische Phase wird mit verdünnter Salzsäure angesäuert und dann nochmals erschöpfend mit MTBE extrahiert. Man erhält 5,8 g eines Öls, das laut 13C-NMR-Spektroskopie zu 80 % aus N-Methoxycarbonyl-2,2-diethylaminoessigsäure (8 % Ausbeute) und zu 20 % aus 2,2-Diethylaminoessigsäure (3% Ausbeute) besteht.

Wie in Beispiel 1 beschrieben, kann aus den Aminosäurederivaten N-Methoxycarbonyl-2,2-diethylaminoessigsäureethylester, 2,2-Diethylaminoessigsäureethylester und N-Methoxycarbonyl-2,2-diethylaminoessigsäure durch Umsetzung mit konzentrierter Salzsäure das Hydrochlorid des Diethylglycins erhalten werden.

### Beispiel 3

In einem Rührgefäß werden 60,0 g (0,32 mol) Cyclopentan-1-carbonsäureethylester-1-carbonsäureamid in 300 g Methanol gelöst. Zu der Lösung, die auf 5 °C abgekühlt worden ist, gibt man 64,0 g (0,32 mol) 20 %ige Natronlauge und 264,5 g (0,33 mol) 9,3 % ige Natriumhypochloritlösung derart hinzu, daß die Temperatur des Reaktionsgemisches 10 °C nicht übersteigt. Man läßt dann 1h bei ca. 5 °C rühren und dosiert anschließend die erhaltene Suspension (während der Chlorierung fällt anorganisches Salz aus) kontinuierlich über eine Fritte, um das Salz abzutrennen. Das klare Filtrat wird kontinuierlich durch einen auf 68 °C beheizten Rohrreaktor dosiert. Man wählt das Volumen des Rohrreaktors und die Dosiermenge pro Zeit so, daß im Rohrreaktor eine Verweilzeit des Filtrats von 2 bis 3 Minuten gewährleistet ist. Nachdem das Filtrat durch den Reaktor dosiert worden ist, wird noch 200 ml Methanol hindurchgepumpt. Der Reaktionsaustrag wird bei einem Druck von 40 mbar und einer Temperatur von 40 °C vom Methanol befreit. Der Rückstand wird erschöpfend mit MTBE extrahiert. Die organischen Phasen werden vereinigt und getrocknet. Anschließend wird das Lösungsmittel abdestilliert. Zurück bleiben 7,1 g eines Öls, das laut 13C-NMR-Spektrum zu >97 % aus N-Methoxycarbonyl-1-aminocyclopentancarbonsäureethylester (Ausbeute: 10 %) besteht.

Die nach der Extraktion zurückbleibende wäßrige, alkalische Phase wird mit verdünnter Salzsäure angesäuert und dann nochmals erschöpfend mit MTBE extrahiert. Man erhält 43,0 g eines Öls, das laut 13C-NMR-Spektroskopie zu 97 % aus N-Methoxycarbonyl-1-aminocyclopentancarbonsäure (70% Ausbeute) besteht. Wie in Beispiel 1 beschrieben, kann aus den Aminosäurederivaten N-Methoxycarbonyl-1-aminocyclopentancarbonsäureethylester und N-Methoxycarbonyl-1-aminocyclopentancarbonsäure durch Umsetzung mit konzentrierter Salzsäure das Hydrochlorid der 1-Aminocyclopentancarbonsäure erhalten werden.

### Beispiel 4

In einem Rührgefäß werden 20,0 g (0,11 mol) t-Butylmalonsäuremonoethylesteramid in 112 g Methanol gelöst. Zu der Lösung, die auf 5 °C abgekühlt worden ist, gibt man 22,0 g (0,10 mol) 20 % ige Natronlauge und 86,0g (0,10 mol) 9,3 % ige Natriumhypochloritlösung derart hinzu, daß die Temperatur des Reaktionsgemisches 10 °C nicht übersteigt. Man läßt dann 3h bei ca. 5 °C rühren und dosiert anschließend die erhaltene Suspension (während der Chlorierung fällt anorganisches Salz aus) kontinuierlich über eine Fritte, um das Salz abzutrennen. Das klare Filtrat wird kontinuierlich durch einen auf 66 °C beheizten Rohrreaktor dosiert. Man wählt das Volumen des Rohrreaktors und die Dosiermenge pro Zeit so, daß im Rohrreaktor eine Verweilzeit des Filtrats von 2 bis 3 Minuten gewährleistet ist. Nachdem das Filtrat durch den Reaktor dosiert worden ist, wird noch 200 ml Methanol hindurchgepumpt. Der Reaktionsaustrag wird bei einem Druck von 40 mbar und einer Temperatur von 40 °C vom Methanol befreit. Der Rückstand wird erschöpfend mit MTBE extrahiert. Die organischen Phasen werden vereinigt und getrocknet. Anschließend wird das Lösungsmittel abdestilliert. Zurück bleiben 9,7 g eines Öls, das laut 13C-NMR-Spektrum zu >98 % aus N-Methoxycarbonyl-t-butylaminoessigsäureethylester (40 % Ausbeute) besteht.

Die nach der Extraktion zurückbleibende wäßrige, alkalische Phase wird mit verdünnter Salzsäure angesäuert und dann nochmals erschöpfend mit MTBE extrahiert. Man erhält 10,4 g eines Feststoffs (Schmp.: 98-100 °C), der laut 13C-NMR-Spektroskopie zu > 98 % aus N-Methoxycarbonyl-t-butylaminoessigsäure (49 % Ausbeute) besteht.
Wie in Beispiel 1 beschrieben, kann aus den Aminosäurederivaten N-Methoxycarbonyl-t-butylaminoessigsäureethylester und N-Methoxycarbonyl-t-butylaminoessigsäure durch Umsetzung mit konzentrierter Salzsäure das Hydrochlorid des tert-Leucins erhalten werden.

### Beispiel 5

In einem Rührgefäß werden 20 g (0,093 mol) 2,2-Dipropylmalonsäuremonoethylesteramid in 50 g Methanol gelöst. Zu der Lösung, die auf 5 °C abgekühlt worden ist, gibt man 18,6 g (0,092 mol) 20 % ige Natronlauge und 87,8 g (0,11 mol) 9,3 % ige Natriumhypochloritlösung derart hinzu, daß die Temperatur des Reaktionsgemisches 10 °C nicht übersteigt. Man läßt dann 1h bei ca. 5 °C rühren und dosiert anschließend kontinuierlich innerhalb von 10 Minuten das erhaltene Reaktionsgemisch unter Rühren in ein auf 60 °C temperiertes Gemisch aus 24,6 g (0,23 mol) Benzylamin und 9,0 g Methanol. Nach der Zudosierung wird das Vorchlorierungsgefäß mit 15,0 g Methanol gespült und die erhaltene Lösung ebenfalls dem Benzylamin/ Methanol - Gemisch zugefügt. Man läßt noch 0,5 h bei 65 °C nachreagieren. Der Reaktionsaustrag wird bei einer Temperatur von 40 °C vom Methanol befreit. Der Rückstand wird erschöpfend mit MTBE extrahiert. Die organischen Phasen werden vereinigt und getrocknet. Anschließend wird das Lösungsmittel abdestilliert. Zurück bleiben 40,3 g eines Öls. Dieses Öl wird in MTBE aufgenommen und die erhaltene Lösung mit verdünnter Salzsäure extrahiert, um den Benzylamin-Überschuß zu entfernen. Die MTBE-Lösung wird mit Wasser nachgewaschen und dann getrocknet. Nach der destillativen Entfernung des MTBE bleiben 23,7 g eines Öls zurück, aus dem ein Feststoff auskristallisiert. Das Gemisch wird in Cyclohexan aufgenommen, der Feststoff durch Filtration abgetrennt und mit wenig Cyclohexan gewaschen. Man erhält 22,8 g Feststoff mit einem Schmelzpunkt von 80 - 83 °C. Es handelt sich um N-2-(2-Propylpentansäure)-N'-benzylharnstoff. Die Ausbeute beträgt 84 %.

## Patentansprüche

1. Verfahren zur Herstellung von Aminosäurederivaten der allgemeinen Formel I 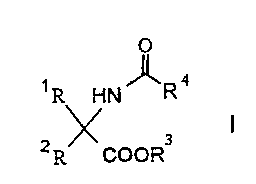 wobei R¹ C₁₋₆-Alkyl oder C₃₋₆-Cycloalkyl und R² C₁₋₆-Alkyl, Phenyl, Heteroaryl-, (CH₂)₁₋₃-Phenyl oder -(CH₂)₁₋₄-COOR bedeutet oder R¹ und R² zusammen - -(CH₂)₂₋₆, -(CH₂)₂-O-(CH₂)₂- oder 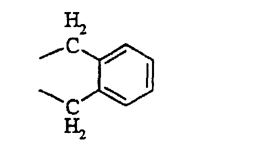 bedeuten,
oder R¹ Wasserstoff und R² einen tertiären Kohlenwasserstoffrest mit 4 bis 10 C-Atomen bedeuten,
R³ Wasserstoff oder einen Alkylrest mit 1-4, insbesondere 1 oder 2 Kohlenstoffatomen und R⁴ OR, wobei R in R² und R⁴ für einen aliphatischen, aromatischen oder araliphatischen Rest mit 1 bis 8 C-Atomen steht, oder NR'R", wobei R' für Wasserstoff oder für einen aliphatischen, aromatischen oder araliphatischen Rest mit 1 bis 8 C-Atomen und R" für Wasserstoff oder einen gegebenenfalls von R' verschiedenen aliphatischen, aromatischen oder araliphatischen Rest von 1 bis 8 C-Atomen steht, bedeutet,
aus den entsprechenden Malonsäuremonoesteramiden der allgemeinen Formel II 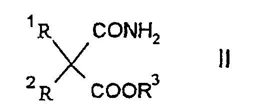 wobei R¹, R² und R³ die oben angegebenen Bedeutungen haben, durch Hofinannschen Abbau mittels eines Hypohalits in wässrig basischem Milieu,
**dadurch gekennzeichnet,**
**daß** man die Reaktion in Gegenwart eines Alkohols oder Amins durchführt.

2. Verfahren nach Anspruch 1,
**dadurch gekennzeichnet,**
**daß** man das Hypohalit in Mengen von 1,0 bis 1,5 Äquivalenten und die Base in Mengen von 0,8 bis 4,0 Äquivalenten je mol Edukt II anwendet.

3. Verfahren nach Anspruch 1,
**dadurch gekennzeichnet, daß** man Alkohole mit 1 bis 8 C-Atomen oder Amine mit 1 bis 16 C-Atomen verwendet.

4. Verfahren nach Anspruch 1,
**dadurch gekennzeichnet, daß** man das Hypohalit in einer Menge von 1,0 bis 1,5 Äquivalenten und die Base in Mengen von 0,8 bis 1,5 Äquivalenten je mol Edukt II anwendet.

5. Verfahren nach Anspruch 1 oder 2,
**dadurch gekennzeichnet,**
**daß** man als Hypohalit ein Alkalihypochlorit und als Base ein Alkalihydroxid verwendet.

6. Verfahren nach einem der Ansprüche 1 bis 4,
**dadurch gekennzeichnet,**
**daß** das Alkalihypochlorit Natriumhypochlorit und das Alkalihydroxid Natriumhydroxid ist.

7. Verfahren nach einem der vorhergehenden Ansprüche,
**dadurch gekennzeichnet, daß** man das Verfahren diskontinuierlich durchführt.

8. Verfahren nach einem der vorhergehenden Ansprüche,
**dadurch gekennzeichnet, daß** man das Verfahren kontinuierlich durchführt

9. Verfahren nach einem der vorhergehenden Ansprüche,
**dadurch gekennzeichnet,**
**daß** man das Malonsäuremonoesteramid zunächst in Gegenwart einer Hypochloritlösung bei 0-20 °C 0,5 bis 4 Stunden lang umsetzt und dann nach Zugabe des Alkalihydroxids bei einer Temperatur von 40 bis 100 °C in einen Alkohol oder ein Amin gegebenenfalls in einem weiteren Lösungsmittel einträgt.

10. Verfahren nach einem der vorhergehenden Ansprüche,
**dadurch gekennzeichnet,**
**daß** man das Malonsäuremonoesteramid in Gegenwart einer Hypochloritlösung und Alkalihydroxydlösung bei 0-20 °C 0,5 bis 4 Stunden lang umsetzt und dann bei einer Temperatur von 40 bis 100 °C in einen Alkohol oder ein Amin gegebenenfalls in einem weiteren Lösungsmittel einträgt.

## Claims

1. A process for the preparation of amino acid derivatives of the general formula I 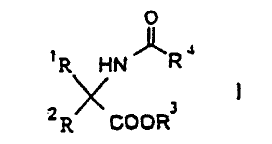 where R¹ is C₁₋₆-alkyl or C₃₋₆-cycloalkyl and R² is C₁₋₆-alkyl, phenyl, heteroaryl-, (CH₂)₁₋₃-phenyl or -(CH₂)₁₋₄-COOR, or R¹ and R² together are -(CH₂)₂₋₆, -(CH₂)₂-O-(CH₂)₂- or 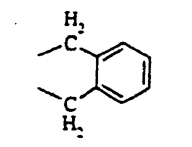 or R¹ is hydrogen and R² is a tertiary hydrocarbon radical having from 4 to 10 carbon atoms,
R³ is hydrogen or an alkyl radical having 1-4, in particular 1 or 2, carbon atoms and R⁴ is OR, where R in R² and R⁴ is an aliphatic, aromatic or araliphatic radical having from 1 to 8 carbon atoms, or NR'R", where R' is hydrogen or an aliphatic, aromatic or araliphatic radical having from 1 to 8 carbon atoms and R" is hydrogen or an aliphatic, aromatic or araliphatic radical having from 1 to 8 carbon atoms which may or may not be different from R',
from the corresponding malonic acid monoester amides of the general formula II 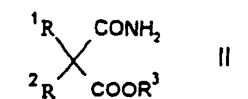 where R¹, R² and R³ are as defined above, by Hofmann degradation using a hypohalite in an aqueously basic medium, **characterized in that** the reaction is carried out in the presence of an alcohol or amine.

2. A process according to claim 1,
**characterized in that**
the hypohalite is used in amounts of from 1.0 to 1.5 equivalents and the base is used in amounts from 0.8 to 4.0 equivalents per mole of starting material II.

3. A process according to claim 1,
**characterized in that** alcohols having from 1 to 8 carbon atoms or amines having from 1 to 16 carbon atoms are used.

4. A process according to claim 1,
**characterized in that** the hypohalite is used in an amount of from 1.0 to 1.5 equivalents and the base is used in amounts of from 0.8 to 1.5 equivalents per mole of starting material II.

5. A process according to claim 1 or 2,
**characterized in that**
the hypohalite used is an alkali metal hypochlorite, and the base used is an alkali hydroxide.

6. A process according to any one of claims 1 to 4,
**characterized in that**
the alkali metal hypochlorite is sodium hypochlorite, and the alkali metal hydroxide is sodium hydroxide.

7. A process according to any one of the preceding claims,
**characterized in that** the process is carried out discontinuously.

8. A process according to any one of the preceding claims,
**characterized in that** the process is carried out continuously.

9. A process according to any one of the preceding claims,
**characterized in that** the malonic acid monoester amide is firstly reacted in the presence of a hypochlorite solution at 0-20°C for from 0.5 to 4 hours and then, after addition of the alkali metal hydroxide, is introduced at a temperature of from 40 to 100°C into an alcohol or an amine, optionally in another solvent.

10. A process according to any one of the preceding claims,
**characterized in that** the malonic acid monoester amide is reacted in the presence of a hypochlorite solution and alkali metal hydroxide solution at 0-20°C for from 0.5 to 4 hours and then, at a temperature of from 40 to 100°C, introduced into an alcohol or an amine, optionally in another solvent.

## Revendications

1. Procédé de préparation de dérivés d'aminoacides de formule générale I 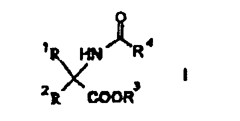 dans laquelle R¹ signific un alkyle en C₁-C₆ ou un cycloalkyle en C₃-C₆ et R² signifie un alkyle en C₁-C₆, un phényle, un hétéroaryle, un (CH₂)₁₋₃-phényle, ou un -(CH₂)₁₋₄-COOR, ou R¹ et R² ensemble signifient (CH₂)₂₋₆, -(CH₂)₂-O-(CH₂)₂ ou 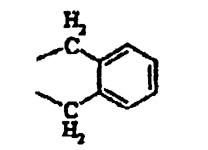 ou R¹ signifie de l'hydrogène et R² un reste hydrocarboné tertiaire ayant de 4 à 10 atomes de carbone, R³ signifie de l'hydrogène ou un reste alkyle ayant de 1 à 4, en particulier 1 ou 2 atomes de carbone, et R⁴ signifie OR dans lequel R dans R² et R⁴, représente un reste aliphatique, aromatique ou arylaliphalique ayant de 1 à 8 atomes de carbone, ou bien NR'R" dans lequel R' représente de l'hydrogène ou un reste aliphatique, aromatique ou arylaliphatique ayant de 1 à 8 atomes de carbone, et R" représente de l'hydrogène ou un reste aliphatiquc, aromatique ou arylaliphatique éventuellement distinct de R', ayant de 1 à 8 atomes de carbone,
à partir des amides monoester d'acide malonique correspondant de formule générale II, 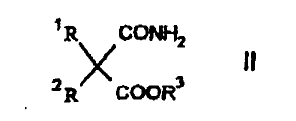 dans laquelle R¹, R² et R³ ont. les significations indiquées ci-dessus, par une dégradation d'Hoffmann à l'aide d'un hypohalite en milieu aqueux basique,
caraetérisé en ce qu'
on effectue la réaction en présence d'un alcool ou d'une aminé.

2. Procédé selon la revendication 1,
**caractérisé en ce qu'**
on utilise l'hypohalite en quantité de 1,0 à 1,5 équivalents et la base en quantités de 0,8 à 4,0 équivalents par mol d'éduit II.

3. Procédé selon la revendication 1,
**caractérisé en ce qu'**
on utilise des alcools ayant de 1 à 8 atomes de carbone ou des amines ayant de 1 à 16 atomes de carbone.

4. Procédé selon la revendication I,
**caractérisé en ce qu'**
on utilise l'hypuhalite en une quantité de 1,0 à 1,5 équivalents et la base en quantités de 0,8 à 1,5 équivalents par mol d'éduit II.

5. Procédé selon l'une quelconque des revendications 1 ou 2,
**caractérisé en ce qu'**
on utilise comme hypohalite un hypochlorite de métal alcalin et comme base un hydroxyde de métal alcalin.

6. Procédé selon l'une quelconque des revendications 1 à 4,
**caractérisé en ce que**
l'hypochlorite de métal alcalin est l'hypochlorite de sodium et l'hydroxyde de métal alcalin est l'hydroxyde de sodium.

7. Procédé selon l'une quelconque des revendications précédentes,
**caractérisé en ce qu'**
on effectue le procédé d'une manière discontinue.

8. Procédé selon l'une quelconque des revendications précédentes,
**caractérisé en ce qu'**
on exécute le procédé en continu,

9. Procédé selon l'une quelconque des revendications précédentes,
**caractérisé en ce qu'**
on fait réagir en premier lieu le monoester amide d'acide malonique en présence d'une solution d'hypochlorite à 0-20°C de 0,5 jusqu'à 4 heures durant, et ensuite après addition de l'hydroxyde de métal alcalin on l'introduit à une température de 40 à 100°C dans un alcool ou une amine, le cas échéant dans un autre solvant.

10. Procédé selon l'une quelconque des revendications précédentes,
**caractérisé en ce qu'**
on fait réagir durant 0,5 à 4 heures à 0-20°C le mnnoester amide d'acide malonique en présence d'une solution d'hypochlorite et d'une solution d'hydroxyde de métal alcalin et ensuite on l'introduit à une température de 40 à 100°C dans un alcool ou unc amine, le cas échéant dans un autre solvant.
